# EUROPEAN PATENT APPLICATION

(11) **EP 1 034 791 A1**
(43) Date of publication of application: **13.09.2000**
(21) Application number: 97954965.6
(22) Date of filing: 28.11.1997
(51) Int. Cl.: A61K 38/43

(54) **ANTI-HYPOXIC AND ANTI-ISCHEMIC DRUG**

(71) Applicant: Karsanov, Nikolai Vasilievich, Moskovskaya obl., Mytischi 141000 (RU)
(72) Inventor: KARSANOV, Nikolai Vasilievich, Moskovskaya obl., Mytischi, 141000 (RU); SUKOYAN, Galina Viktorovna, Moskovskaya obl., Mytischi, 141000 (RU); TATULASHVILI, Dali Robertovna, Tbilisi, 380060 (GE); KARSANOV, Zakhary Nikolaevich, Tbilisi, 380060 (GE); KARSANOV, Vasily Nikolaevich, Moskovskaya obl., 142292 (RU)
(74) Representative: Grussdorf, Jürgen, Dr.
(86) International application number: RU9700383
(87) International publication number: WO9927952

(57) **Abstract**

The invention relates to the field of medicine and more particularly to novel drugs which improve the resistance of human organism and tissues to hypoxia and ischemia by preventing and blocking homeostasis disorders of the energy system and allowing full recovery of the homeostasis pathologic condition in case of ischemic and hypoxic lesions of the cell. According to the invention, an anti-hypoxic and anti-anginal drug contains a metabolic ingredient (Energostim) and coferments such as flavin adenine dinucleotide (or its precursor flavin mononucleotide or riboflavin mutually transforming in the organism or a derived product of riboflavin, the benzoflavin, which is also transformed into desired flavins) as well as the coferment Q₁₀ (the ubiquinone). The drug has the following composition: coferments: 1,2 -20 %; metabolic ingredient: 65,5 - 98,8 %. The drug of this invention may be-widely used to strengthen the resistance of the human body and tissues to the hypoxia, to improve the working capacity in extreme conditions, in the space and in deep water as well as for the treatment of brain ischemia and of myocardial infarction and of other ischemic and hypoxic conditions of tissues and organs.

## Description

### Field of the invention

The present invention relates to the field of medicine and is particularly concerned with the creation of drugs which increase the tolerance of the body, organs and tissues to hypoxia and ischemia by preventing and aborting the progress of homeostatic disturbancies of the energy supply system and by allowing full restoring of the homeostatic pathologic condition in case of ischemic and hypoxic damage of the cell.

### Background of the invention

Known in the art is an antiischemic and antihypoxic preparation "**Energostim**" (RU patent 2035907). This metabolic preparation contains 0,5 mg of nicotinamide adenine dinucleotide (NAD), 10 mg of cytochrome C and 80 mg of inosine which is the metabolic product of degradation of adenyl nucleotides.

In antihypoxic action Energostim surpasses all presently known drugs many times. For example, the action of Energostim is 7,5-fold more effective than that of γ-aminobutyric acid (GABA), which is the most efficient antihypoxic drug, and 5,7-fold more effective than that of γ-oxibutyric acid (GOBA). Such a high efficacy of Energostim is caused by the fact that it contains nicotinamide adenine dinucleotide (NAD) which is the main acceptor of reduction equivalents. Energostim makes up for the low concentration of NAD under hypoxia and thus restores the normal course of redox-reactions in the cytosol and mitochondria, where NAD-dependent dehydrogenases take part. In addition, Energostim activates the transport of a proton from cytosol into mitochondria via malate-aspartate shunt, and the proton further proceeds to oxygen on the electron transfer chain. In the end the exogenous NAD strengthens the ATP synthesis under hypoxia by way of glycolysis, and during the process of oxidative phosphorylation the energy supply system in mitochondria regains its ability to respond to regulatory actions.

The second ingredient of Energostim, i.e.. cytochrome C, is built into the cytochrome C-deficient membrane of mitochondria under hypoxia and increases the transport (flow) of electrons to oxygen as well as the ATP synthesis in the oxidative phosphorylation reaction coupled with electron transport.

Inosine, the third ingredient of **Energostim**, stimulates the synthesis of adenyl nucleotides *de novo* and intensifies the blood circulation which also promotes the ATP synthesis. In addition inosine increases immune system activity of the body.

Individually none of the ingredients of **Energostim** can be as effective (both qualitatively and quantitatively) as the combined preparation, because restoration of only one link in a complicated system of energy supply does not lead to restoration of another and for this reason it cannot result in the overall positive effect of the combined preparation as well as cannot restore a normal regulation of processes that bring about formation of easily available energy in the cell.

However, **Energostim** does not restore the homeostasis of energy supply system of the cell in full measure.

The object of the present invention is to work out a combined preparation with such a set of ingredients which ensures a stable raising of protective capacity of the body against hypoxia, increase of the body reserve possibilities and full restoration of the homeostatic state of the energy supply system in cell under hypoxic damages.

### Diclosure of the invention

This object ist attained in an antihypoxic and antiischemic drug based on a metabolic component (Energostim) by additionally incorporating therein coenzymes: flavin adenine dinucleotide (FAD), flavin mononucleotide (FMN) or their precursor riboflavin, all of them being interconverted in the body, or its derivative benzoflavin, which also transforms in the cell into flavins necessary for the cell, as well a coenzyme Q₁₀ (ubiquinone), the ratio of the component being as follows (parts by weight):

| | |
|---|---|
| coenzymes | 1,2 - 20, |
| metabolic component | 65,5 - 98,3. |

Flavin adenine dinucleotide (FAD), which is the second to NAD main electron carrier under oxidation of fuel molecules, accepts, through its isoalloxazine ring (riboflavin), two electrons and both protons lost by the substrat (as opposed to NAD, which adds only one of the two liberated protons). FAD is the only hydrogen acceptor at the stage of succinate-to-fumarate convertion, since the change in free energy at this stage is not enough for reduction of NAD. It has been shown that in the cycle of tricarboxylic acids for three molecules of NAD there is one molecule of FAD. In this connection it is an easy matter to imagine the magnitude of the effect arising from the drop of FAD content under hypoxic conditions such as ischemia [*T.V. Fetisova, P.A. Frolkis. Biochemistry of myocardial infarction Kiev. "Zdorovia" 1976*]. ( **FAD** also plays a role of prosthetic group of enzymes at the forth concluding stage of Krebs cycle with NAD being oxidant. FAD and its degradation product FMN play an important specific role at various stages of Krebs cycle and the electron transfer process. Particularly, during the process of oxidative phosphorilation the electrons are first carried from NADH onto FMN, the latter forming the prosthetic group of NADH-dehydrogenase, and only after that moment they pass through iron-sulfur complexes and coenzyme Q₁₀ and are then carried over the electron tranferring cytochrome chain to oxygen.

The coenzyme Q₁₀ is a highly mobile carrier of electrons from flavinoproteides to the chain of cytochromes. Thus, the coenzyme Q₁₀ fulfils a collector function gathering pairs of reduction equivalents that come from various substrats. Consequently, NAD collects reduction equivalents from NAD- and NADRN-dependent substrats being affected by flavin-dependent dehydrogenases. At this takes place, ubiquinone ist the single carrier of the respiratory chain, which is linked to protein unstably, noncovalently. This accounts for its easily being washed out from the membranes of cell mitochondria when the cell is damaged.

Consequently, the incorporation of coenzyme FAD (it converts during the process of degradation into other flavins indispensable for the cell) and conenzyme Q₁₀ provides a complex antihypoxic drug which is capable to restore homeostasis of the energy supply system and, as a result, to increase its reserve potential, to raise the resistance of the cell and the body as a whole to hypoxia and to protect it against a hypoxic damage.

Glucose is proposed to be advantageously used as a solvent whereby the process of glycolysis is to be provided with exogenous substrat (under ischemic and hypotoxic conditions the glycogen content of tissue decreases).

The basis for the action of the present combined preparation hereinafter referred to as Energostim-Neo resides in harmonical impact of all the ingredients (rather than in a mere summation of component effects) whereby the preparation is given quite a new property possessed by none of the ingredients alone. By simultaneously affecting the key links of the energy supply system, Energostim-Neo prevents the disturbance of homeostasis of the system and brings about its rapid restoration.

FAD (0,3 mg/kg) or FMN (0,6 mg/kg) or riboflavin (2 mg/kg) or benzoflavin (5 mg/kg), coenzyme Q₁₀ (2 mg/kg), NAD (0,5 mg/kg), cytochrome C (10 mg/kg), inosine (80 mg/kg) were mixed and dissolved immediately before use at a temperature of 25 to 35°C and thoroughly agitated in a 40 percent glucose solution or a saline solution. The preparation was made in the dark because FAD is a light-sensitive substance.

### Example 1.

The study was carried out on white hybrid rats, each weighing 200 to 250 g. The rats were previously divided into two groups of those with low resistance and those with high resistance to acute oxygen insufficiency.

The antihypoxic activity of the combined drug, its prior art and ingredients as well as some standard antihypoxants were studied on the model of acute hypobaric hypoxia, complicated with hypercapnia - the animals were taken up to the altitude of 11,5 thousand meters at a speed of 50 m/s in a closed space without pumping out CO₂.
All the experimental animals were divided into equal groups with 7 rats in each. The first group was made up of the animals which received the developed
NAD, cytochrome C, FAD, riboflavin, FMN, benzoflavin, coenzyme Q10 and inosine respectively, 11 - GOBA (gamma-oxybutyric acid), 12 ― GABA (gamma-aminobutyric acid), 13 ― Energostim-Neo + GOBA, 14, 15 ― Energostim-Neo + GABA (GABA or GOBA are drugs widely used as a standard antihypoxic preparation), 13 ― guthimine, 14 ―α-tocopherol. The respective antihypoxant was administered intraperitoneally 15 and 40 minutes before lifting in the pressure chamber. Time of survival (Ts) (reserve time in min) "at height" to the coming of the second agonal inhalation was registrated.

The results of investigation are shown in the table 1.

They demonstrate that Energostim-Neo increases the Ts value in the group of animals with low resistance to hypoxia (with the same quantitative contents of NAD, cytochrome C and inosine as in Energostim) 1,5-fold as compared with Energostim and 8,6-fold as compared with GOBA, and is 6,6 times more effective than the total effect of the ingredients taken alone (see table). This amounts to the increase in the hypoxia protection coefficient for Energostim-Neo 39,5-fold, for Energostim 26,5-fold and for GOBA only 4,6-fold, all the preparations of the remainder and their combinations giving an increase of about 2 to 3-fold (the preparation is deemed to be antihypoxant if it improves the survival time (Ts) at least twofold). It is interesting to note that the combination of Energostim-Neo with GOBA or GABA fails to increase antihypotoxic properties, it rather decreases the protection coefficient as compared with pure Energostim-Neo. It should be noted that the effect of Energostim-Neo in respect to both indices is more pronounced than the that of Energostim.

Increasing the concentration of flavin adenine dinucleotide up to 3,0 mg/kg and coenzyme Q up to 10 mg/kg in Energostim-Neo or, to the contrary, decreasing their contents down to 0,1 and 1,0 mg/kg respectively diminishes the Ts value to 45,5 min (about 1,5-fold).

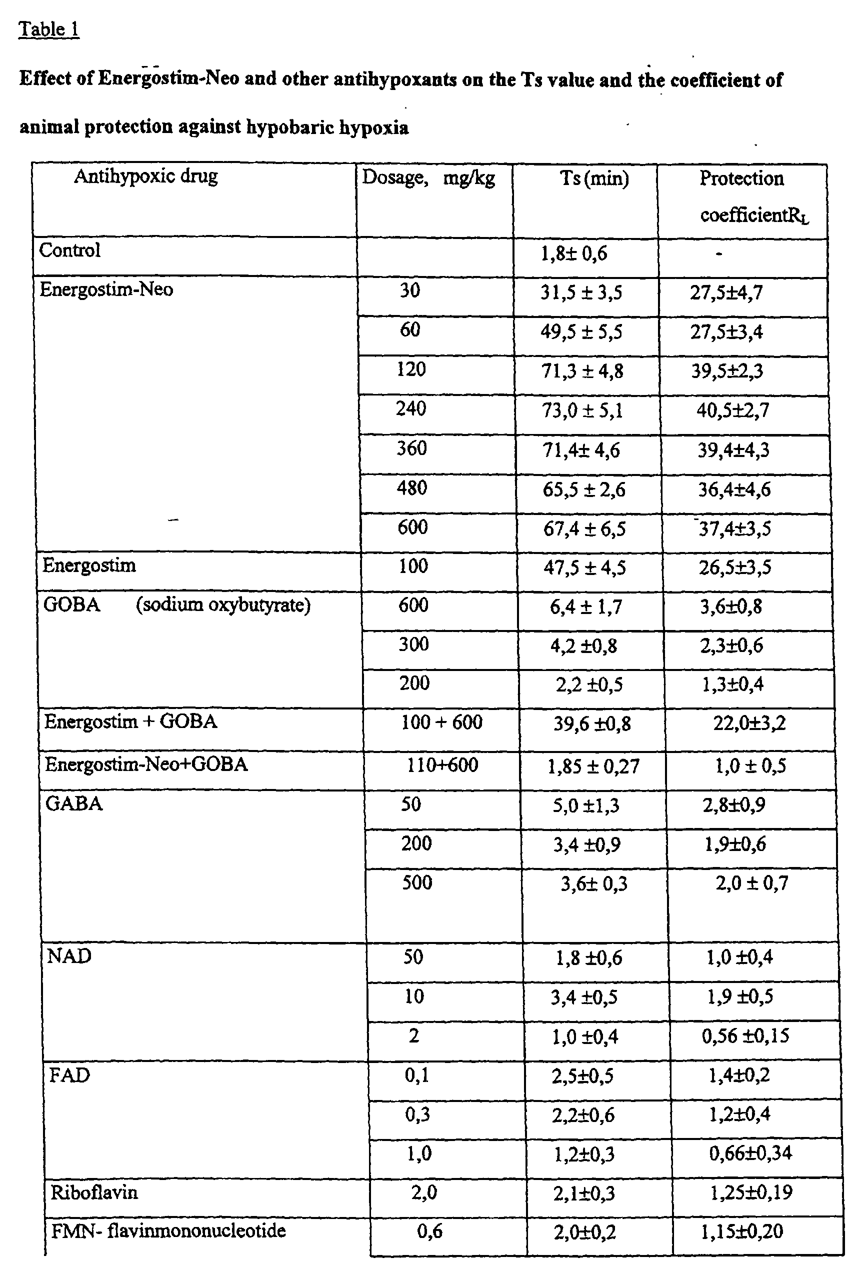

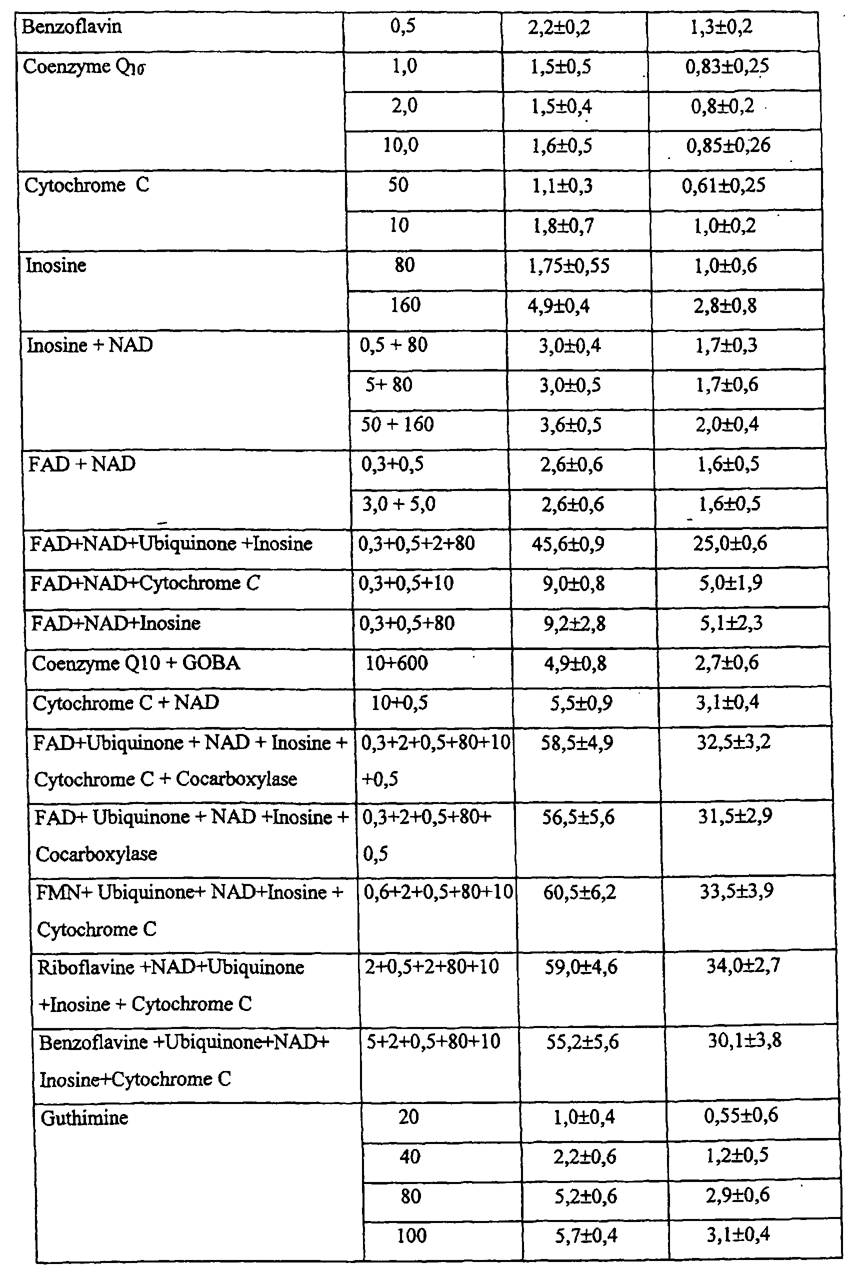

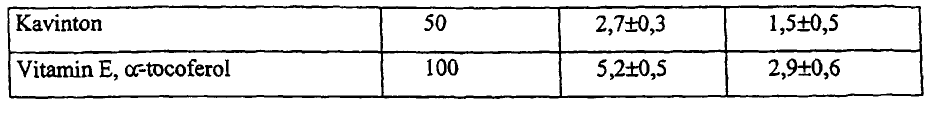

Thus, the present drug, Energostim―Neo, which should be regarded in practical use as a low toxic protective antihypoxic drug and a remedy of replacement therapy, opens quite new possibilities in enhancing the resistance (protection) of the human being against hypoxia and new approaches in treatment of hypoxic and ischemic conditions.

In the state of alcoholic damage of myocardium when with the decrease of NAD and cytochrome C in the cell (N.V. Karsanov et al.,1995) the content of thiamine also diminishes at the same time, this combination, intended for the treatment of a hypoxic damage resulting from alcoholic intoxication, has been strengthened with cocarboxylase. Cocarboxylase directly takes part in activation of pyruvate decarboxylase in the reaction of conversion of pyruvate into acetaldehyde whose subsequent destruction takes place with the cooperation of NAD-dependent acetaldehyde dehydrogenase.

### Industrial applicability

It is suggested that present drug will be widely used to increase tolerance of the human body and tissue to hypoxia, working capacity under extreme conditions in space and at great depths as well as in treatment of ischemia of brain, myocardium infarction and other ischemic and hypoxic states of tissues and organs.

## Claims

1. Antihypoxic and antianginal drug, based on a metabolic component characterized in that it additionally contains coenzymes with the following ratio of components (in parts by weight):
| | |
|---|---|
| coenzymes | 1,2 to 20; |
| metabolic component | 65,5 to 98,7. |

2. Antihypoxic and antianginal drug according to claim 1, characterized in that as the metabolic component it contains (in parts by weight):
| | |
|---|---|
| nicotinamide adenine dinucleotide | 0,5 to 10; |
| cytochrome C | 5,0 to 20; |
| inosine | 65,0 to 92,2. |

3. Antihypoxic and antianginal drug according to claim 1, characterized in that as the metabolic component it contains (in parts by weight):
| | |
|---|---|
| nicotinamide adenine dinucleotide | 0,5 to 20; |
| inosine | 65,0 to 98,3. |

4. Antihypoxic and antianginal drug according to claim 1, characterized in that as the coenzymes it contains (in parts by weight):
| | |
|---|---|
| flavin | - 0,2 to 10; |
| coenzyme Q₁₀ | - 1,0 to 10. |

5. Antihypoxic and antianginal drug according to claim 4, characterized in that as flavine it contains 0,2 to 2 parts by weigth of flavin adenine dinucleotide.

6. Antihypoxic and antianginal drug according to claim 4, characterized in that as flavin it contains 0,2 to 10 parts by weigth of flavin mononucleotide.

7. Antihypoxic and antianginal drug according to claim 4, characterized in that as flavin it contains 0,5 to 10 parts by weigth of riboflavin.

8. Antihypoxic and antianginal drug according to claim 4, characterized in that as flavin it contains 2 to 10 parts by weigth of benzoflavin.

9. Antihypoxic and antianginal drug according to claim 1, characterized in that as the metabolic component it contains (in parts by weight):
| | |
|---|---|
| nicotinamide adenine dinucleotide | 0,5 to 1.5; |
| inosine | 65,0 to 91,6; |
| cytochrome C | 5 to 18; |
| cocarboxylase | 0,1 to 2. |

10. Antihypoxic and antianginal drug according to claim 1, characterized in that the metabolic component contains (in parts by weigth):
| | |
|---|---|
| nicotinamide adenine dinucleotide | 0,5 to 10; |
| inosine | 65,0 to 93,3; |
| cocarboxylase | 0,1 to 2. |
